# EUROPEAN PATENT APPLICATION

(11) **EP 3 809 114 A1**
(43) Date of publication of application: **21.04.2021**
(21) Application number: 19819178.5
(22) Date of filing: 24.05.2019
(51) Int. Cl.: G01N 15/06, G01N 21/47

(54) **ENVIRONMENT MEASUREMENT DEVICE, INFORMATION PROCESSING DEVICE, INFORMATION PROCESSING SYSTEM, INFORMATION PROCESSING METHOD, AND PROGRAM**

(30) Priority: 13.06.2018 JP 2018112700
(71) Applicant: Sony Corporation, 108-0075 Tokyo (JP)
(72) Inventor: ENOKIDO, Toshio, Tokyo 108-0075 (JP); MIYASHITA, Ken, Tokyo 108-0075 (JP); WAKITA, Yoshihiro, Tokyo 108-0075 (JP); KAWAMOTO, Yohei, Tokyo 108-0075 (JP); AIZAWA, Kota, Tokyo 108-0075 (JP)
(74) Representative: 2SPL Patentanwälte PartG mbB
(86) International application number: PCT/JP2019/020686
(87) International publication number: WO 2019/239855

(57) **Abstract**

[Object] To provide an environmental measurement apparatus that detects a fine particle in the air such as smoke or an odor and can be made small, an information processing apparatus that uses measurement data acquired using the environmental measurement apparatus, an information processing system, an information processing method, and a program.

[Solving Means] An environmental measurement apparatus includes a light source and a light-receiving-element array. The light source irradiates light onto a fine particle in gas. The light-receiving-element array includes a plurality of light receiving elements receiving backscattered light obtained by the light being irradiated onto the fine particle.

## Description

### Technical Field

The present technology relates to an environmental measurement apparatus, an information processing apparatus, an information processing system, an information processing method, and a program.

### Background Art

Smoking may be harmful to others' health due to passive smoking being caused by secondhand smoke. This results in going ahead with separation of smoking and nonsmoking areas. For example, even in a nonsmoking area in a restaurant that has adopted separation of smoking and nonsmoking areas, there may exist a table in which a feeling of being bothered by smoke or an odor of a cigarette is caused, although it depends on where in the restaurant. This may cause the user to feel uncomfortable.

For example, Patent Literature 1 discloses placing, near a pollen-dispersal source, a pollen-particle measurement apparatus that measures a pollen particle that is an example of a fine particle, and calculating information regarding a prediction of pollen dispersal using information regarding an amount of pollen particles that is obtained from the pollen-particle measurement apparatus. The pollen-particle measurement apparatus disclosed in Patent Literature 1 includes an intake device that intakes air, a tank that stores therein the intaken air, an irradiation device that irradiates laser light onto pollen particles in the air in the tank, a detection photodiode that converts a change in the laser light into an electric signal, and a fan used to discharge the air irradiated with the laser light to the outside of the tank.

### Citation List

### Patent Literature

Patent Literature 1: Japanese Patent Application Laid-open No. 2008-216133

### Disclosure of Invention

### Technical Problem

Since the pollen-particle measurement apparatus described above includes the intake device, the tank, the fan, and the like, the entire apparatus is large in size and it is difficult to make the apparatus small. This makes it difficult to carry the apparatus and thus to measure, for example, a fine particle such as cigarette smoke situated around an individual.

In view of the circumstances described above, it is an object of the present technology to provide an environmental measurement apparatus that detects a fine particle in the air such as smoke or an odor and can be made small, an information processing apparatus that uses measurement data acquired using the environmental measurement apparatus, an information processing system, an information processing method, and a program. Solution to Problem

In order to achieve the object described above, an environmental measurement apparatus according to an embodiment of the present technology includes a light source and a light-receiving-element array.

The light source irradiates light onto a fine particle in gas.

The light-receiving-element array includes a plurality of light receiving elements receiving backscattered light obtained by the light being irradiated onto the fine particle.

In such a configuration, a light-receiving-element array including a plurality of light receiving elements is used. Thus, there is no need for the gas of a certain level of fine-particle concentration for measurement of a fine particle. Further, there is also no need to provide an intake device or the like used to intake air. Therefore, it is possible to make the environmental measurement apparatus small.

The environmental measurement apparatus may further include a position-information acquisition section that acquires position information regarding a position of the environmental measurement apparatus; and a communication section that communicates another apparatus, and transmits, to the other apparatus, measurement data measured by the light receiving element and the position information.

Such a configuration makes it possible to transmit, to another apparatus, measurement data measured by the environmental measurement apparatus, and position information regarding a place in which the measurement data has been acquired.

The environmental measurement apparatus may further include a diffraction grating that the light from the light source enters, the diffraction grating emitting the light that is to be irradiated onto the fine particle.

The environmental measurement apparatus may further include a mirror off which the backscattered light is reflected to enter the light-receiving-element array.

The environmental measurement apparatus may further include a plurality of the light-receiving-element arrays; and a mirror through which light of a first wavelength from among the backscattered light is transmitted to enter one of the plurality of the light-receiving-element arrays, the mirror being a mirror off which light of a second wavelength that is different from the first wavelength is reflected to enter another of the plurality of the light-receiving-element arrays.

In order to achieve the object described above, an information processing apparatus according to an embodiment of the present technology includes an acquisition section.

The acquisition section acquires measurement data of a fine particle in gas, and position information regarding a place in which the measurement data has been acquired, the measurement data and the position information being acquired by an environmental measurement section that includes a light source and a light-receiving-element array, the light source emitting light, the light-receiving-element array including a plurality of light receiving elements receiving backscattered light obtained by the emitted light being irradiated onto the fine particle.

In such a configuration, the use of a light-receiving-element array including a plurality of light receiving elements makes it possible to make an environmental measurement section small. This results in being able to provide an apparatus that includes the environmental measurement section and is sufficiently small to be portable. When an individual user has such an apparatus that includes the environmental measurement section and is sufficiently small to be portable, the environmental measurement section moves according to the movement of the user. This enables the information processing apparatus to acquire measurement data of a fine particle around the individual user in various places.

The acquisition section may acquire the pieces of measurement data and the pieces of position information, the pieces of measurement data being respectively acquired by a plurality of the environmental measurement sections, the pieces of position information being respectively acquired by the plurality of the environmental measurement sections.

Such a configuration makes it possible to acquire a plurality of pieces of measurement data of a fine particle in various places from a plurality of environmental measurement sections.

The information processing apparatus may further include a learning section that performs statistical processing with respect to a relationship between the position information and the measurement data that are acquired from each of the plurality of the environmental measurement sections.

Such a configuration makes it possible to obtain information regarding a correlation between position information and measurement data of a fine particle.

The acquisition section may acquire the measurement data of the fine particle and feeling information regarding a feeling of a user with respect to the fine particle upon acquiring the measurement data, and the learning section may perform statistical processing with respect to a relationship between the measurement data obtained from each of the plurality of the environmental measurement sections, and the feeling information.

Such a configuration makes it possible to obtain information regarding a correlation between measurement data of a fine particle and feeling information.

The information processing apparatus may further include a database building section that builds a database by accumulating therein the measurement data and position information regarding a region in which the measurement data has been acquired, the measurement data and the position information regarding the region being accumulated in a state of being associated with each other, the measurement data and the position information regarding the region being acquired by the environmental measurement section.

The database building section may accumulate therein information related to the fine particle, the measurement data, and the position information regarding the region in association with each other, the information related to the fine particle being generated on the basis of the measurement data and the position information regarding the region, and the information processing apparatus may further include an extraction section that refers to the database and extracts information related to a fine particle in gas in an arbitrary place selected by the user, the information related to the fine particle in the gas in the arbitrary place being associated with position information regarding the arbitrary place.

The information processing apparatus may further include an information providing section that provides the information related to the fine particle in the gas in the arbitrary place, the information related to the fine particle in the gas in the arbitrary place being extracted by the extraction section.

Such a configuration makes it possible to provide a user with information related to a fine particle in an arbitrary place selected by the user.

The information providing section may provide the user with the information related to the fine particle in the gas in the arbitrary place, the information related to the fine particle in the gas in the arbitrary place being information in which the feeling information has been considered.

Such a configuration makes it possible to provide information related to a fine particle that is suitable for each user.

In order to achieve the object described above, an information processing system according to an embodiment of the present technology includes an environmental measurement apparatus and an acquisition section.

The environmental measurement apparatus includes a light source and a light-receiving-element array, the light source irradiating light onto a fine particle in gas, the light-receiving-element array including a plurality of light receiving elements receiving the light irradiated onto the fine particle.

The acquisition section acquires a piece of measurement data of the fine particle and a piece of position information regarding a place in which the piece of measurement data has been acquired, the pieces of measurement data and the pieces of position information being acquired by a plurality of the environmental measurement apparatuses.

In order to achieve the object described above, an information processing method according to an embodiment of the present technology includes acquiring a piece of measurement data of a fine particle in gas and a piece of position information regarding a place in which the piece of measurement data has been acquired, the pieces of measurement data and the pieces of position information being acquired by a plurality of environmental measurement sections each including a light source and a light-receiving-element array, the light source irradiating light onto a fine particle in gas, the light-receiving-element array including a plurality of light receiving elements receiving the light irradiated onto the fine particle.

In order to achieve the object described above, a program according to an embodiment of the present technology causes an information processing apparatus to perform a process including acquiring a piece of measurement data and a piece of position information regarding a place in which the piece of measurement data has been acquired, the pieces of measurement data and the pieces of position information being acquired by a plurality of environmental measurement sections each including a light source and a light-receiving-element array, the light source irradiating light onto a fine particle in gas, the light-receiving-element array including a plurality of light receiving elements receiving the light irradiated onto the fine particle.

### Advantageous Effects of Invention

As described above, the present technology makes it possible to make the environmental measurement apparatus small, and thus to measure a fine particle situated around an individual user. Note that the effect described here is not necessarily limitative, and any of the effects described in the present disclosure may be provided.

### Brief Description of Drawings

[Fig. 1] Fig. 1 is a conceptual diagram of an information processing system 1 according to a first embodiment.
[Fig. 2] Fig. 2 is a diagram for describing the information processing system, and is a block diagram illustrating configurations of a cloud server and a cellular phone.
[Fig. 3] Fig. 3 schematically illustrates an environmental measurement section according to the first embodiment.
[Fig. 4] Fig. 4 schematically illustrates a light-receiving-element array included in the environmental measurement section.
[Fig. 5] Fig. 5 illustrates an example of measurement data in a light receiving element included in the light-receiving-element array.
[Fig. 6] Fig. 6 illustrates an example of pieces of measurement data in the light receiving elements included in the light-receiving-element array.
[Fig. 7] Fig. 7 is a diagram describing detection of a movement of a fine particle of cigarette smoke using the example of the pieces of measurement data of Fig. 6.
[Fig. 8] Fig. 8 illustrates an example of a cigarette-smoke interior map.
[Fig. 9] Fig. 9 illustrates an example of building of a database.
[Fig. 10] Fig. 10 is a flowchart describing an information processing method related to provision of a fine particle that is performed in the cloud server according to the first embodiment.
[Fig. 11] Fig. 11 schematically illustrates how a user U starts an application and uses a cigarette-smoke interior map in the information processing system according to the first embodiment.
[Fig. 12] Fig. 12 schematically illustrates an environmental measurement section according to a second embodiment.
[Fig. 13] Fig. 13 schematically illustrates an environmental measurement section according to a third embodiment.
[Fig. 14] Fig. 14 schematically illustrates an environmental measurement section according to a fourth embodiment, and illustrates an example of measurement data.
[Fig. 15] Fig. 15 schematically illustrates an environmental measurement section according to a fifth embodiment.
[Fig. 16] Fig. 16 schematically illustrates how the user U starts an application and uses a cigarette-smoke interior map in the information processing system according to a sixth embodiment.
[Fig. 17] Fig. 17 schematically illustrates how the user U starts an application and uses a park map in the information processing system according to a seventh embodiment.
[Fig. 18] Fig. 18 illustrates examples of images displayed on a cellular phone when the user U starts an application and searches for a place of a favorite odor in the information processing system according to an eighth embodiment.
[Fig. 19] Fig. 19 illustrates examples of images displayed on the cellular phone when the user U starts the application and searches for a place of a favorite odor in the information processing system according to the eighth embodiment.

### Mode(s) for Carrying Out the Invention

Embodiments according to the present technology will now be described below with reference to the drawings.

### (First Embodiment)

### [Conceptual Diagram of Information Processing System]

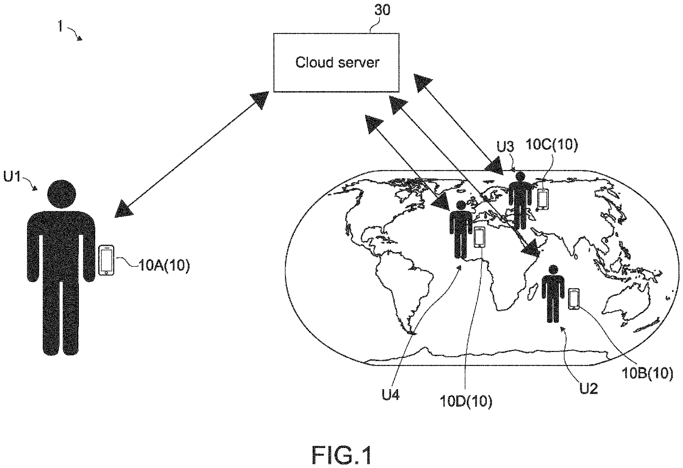

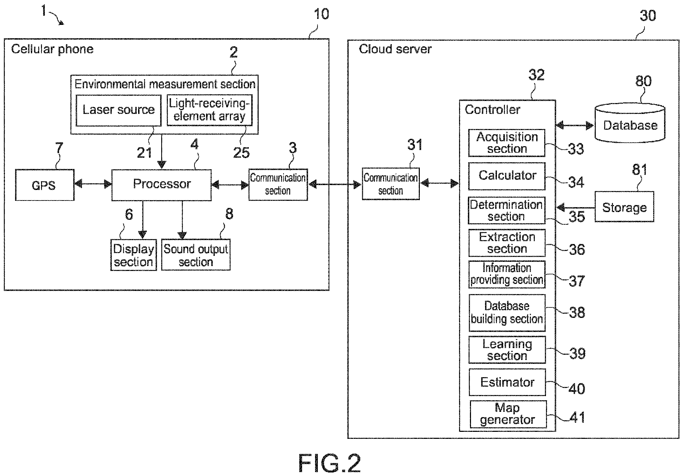

Fig. 1 is a conceptual diagram of an information processing system 1 according to the present embodiment. Fig. 2 is a diagram for describing the information processing system 1, and is a block diagram illustrating configurations of a cloud server that is an information processing apparatus, and a cellular phone.

As illustrated in Figs. 1 and 2, the information processing system 1 includes a cloud server (hereinafter simply referred to as a server) 30, and cellular phones 10A to 10D respectively carried by a plurality of users U1 to U4. The server 30 and the cellular phone 10 are capable of communicating with each other.

Note that, in this case, the number of cellular phones is four for convenience, but the number of cellular phones is not limited to this. Further, the cellular phone is simply referred to as the cellular phone 10 when there is no need to distinguish among, for example, the cellular phones 10A to 10D, and the user is simply referred to as a user U when there is no need to distinguish among, for example, the users U1 to U4.

The cellular phone 10 includes an environmental measurement section 2 and serves as an environmental measurement apparatus that detects a fine particle in ambient air. The cellular phone 10 also serves as an apparatus that receives fine-particle information regarding a fine particle in an arbitrary place, the fine-particle information being provided by the server 30.

The server 30 acquires measurement data of a fine particle measured by the environmental measurement section 2 of each of the cellular phones 10A to 10D, and position information regarding a place in which the fine particle has been measured, and chronologically accumulates, in a database 80, the measurement data and the position information in association with each other. Accordingly, the server 30 builds the database 80.

In accordance with a request from the user U, the server 30 provides, to the cellular phone 10 and on the basis of the database 80, information related to a fine particle in an arbitrary place selected by the user.

In the present embodiment, the description is made using a restaurant as an example of the arbitrary place and a fine particle of cigarette smoke as an example of the fine particle.

### [Configuration of Cellular Phone]

As illustrated in Fig. 2, the cellular phone 10 includes the environmental measurement section 2, a communication section 3, a processor 4, a display section 6, a global positioning system (GPS) 7 that is a position-information acquisition section, and a sound output section 8.

The environmental measurement section 2 includes a laser source 21 and a light-receiving-element array 25. The environmental measurement section 2 detects a fine particle in the air. The cellular phone 10 of the present embodiment is provided with a hole passing through the cellular phone 10 in the thickness direction, and the air passing through the hole is a detection target.

The communication section 3 communicates with the server 30 that is another apparatus. The communication section 3 transmits, to the server 30, measurement data measured by the environmental measurement section 2 and position information acquired by the GPS 7 as position information regarding a place in which the measurement data has been measured.

The communication section 3 receives a cigarette-smoke interior map of a restaurant from the server 30.

The display section 6 includes a display panel made of, for example, a liquid crystal element or an organic electroluminescence (EL) element, and a transparent touch panel bonded to an upper surface of the display panel. The display section 6 displays an image using the display panel, and also serves as an input section on which an input operation can be performed by the user U through the touch panel.

Note that the cellular phone 10 may include a microphone that is an example of a sound input section that collects sound, and may be capable of receiving a sound input.

The GPS 7 acquires position information regarding the cellular phone 10 also serving as an environmental measurement apparatus. The position information regarding the cellular phone 10 is also position information regarding the user U who has the cellular phone 10 by wearing it.

The sound output section 8 is typically a speaker and outputs sound.

The processor 4 receives image-signal information and sound-signal information that are used to notify the user U of information regarding a fine particle that is received from the server 30 through the communication section 3.

Examples of the information regarding a fine particle include a cigarette-smoke interior map of the restaurant that is a fine-particle map of an arbitrary place, and a suggestion generated on the basis of the map.

The processor 4 causes an image to be displayed on the display section 6 on the basis of the received image-signal information, and causes the sound output section 8 to output sound on the basis of the sound-signal information.

Further, the processor 4 transmits, to the server 30 and through the communication section 3, information corresponding to a touch-input operation performed by the user U on the display section 6.

Furthermore, the processor 4 transmits, to the server 30 and through the communication section, the position information acquired by the GPS 7 and the measurement data acquired by the environmental measurement section 2.

An application used to obtain fine-particle information is installed on the cellular phone 10. When the user U starts the application, it is possible to acquire, from the server 30, the fine-particle information regarding cigarette smoke that is a fine particle.

### [Configuration of Environmental Measurement Section]

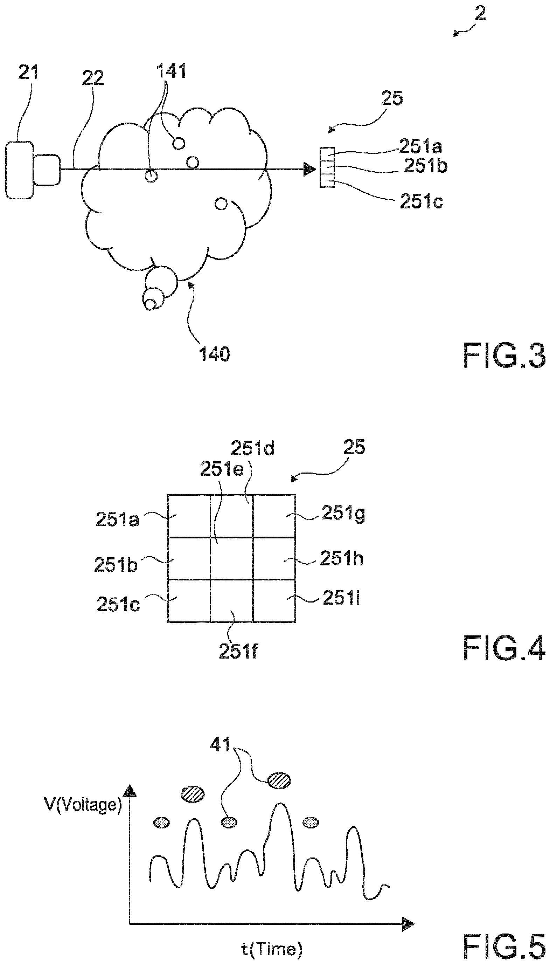

Fig. 3 schematically illustrates the environmental measurement section 2. Fig. 4 schematically illustrates the light-receiving-element array.

As illustrated in Fig. 3, the environmental measurement section 2 includes the laser source 21 emitting laser light 22, and the light-receiving-element array 25. The environmental measurement section 2 measures a fine particle 141 in gas 140 such as cigarette smoke. Typically, highly directional laser light is used to measure the fine particle 141.

The laser source 21 is arranged to be capable of irradiating laser light onto the air passing through a through-hole provided to the cellular phone 10. The light-receiving-element array 25 is arranged at a position at which the light-receiving-element array 25 is capable of receiving backscattered light from the fine particle 141 in the gas 140 such as cigarette smoke, the fine particle 141 in the gas 140 being irradiated with the laser light 22.

The laser source 21 irradiates the laser light 22 onto the fine particle 141 in the gas 140 such as cigarette smoke.

The light-receiving-element array 25 receives scattered light, backscattered light in the present embodiment, that is generated from the fine particle 141 irradiated with the laser light.

The light-receiving-element array 25 includes a plurality of light receiving elements 251 formed of photodiodes. The plurality of light receiving elements 251 includes light receiving elements provided in an (n×m)-arrangement on the same plane, where n and m are integers and at least one of n or m is two or more. For example, a light-receiving-element array including light receiving elements provided in a 10×10-arrangement has a size of about 2 mm × 2 mm. In the present embodiment, an example in which nine light receiving elements 251a to 251i in total are provided in a 3×3-arrangement is described for convenience, as illustrated in Fig. 4. The light receiving element is simply referred to as the light receiving element 251 when there is particularly no need to individually distinguish among the light receiving elements 251a to 251i.

The light receiving element 251 converts detected scattered light into a voltage signal. Measurement data is acquired for each of the light receiving elements 251a to 251i by measurement being performed by the light-receiving-element array 25.

Fig. 5 illustrates an example of measurement data in a certain light receiving element 251, where the horizontal axis indicates time, and the vertical axis indicates voltage.

The measurement data includes information regarding the particle size of the fine particle 141 and information regarding a concentration of the fine particle 141. As illustrated in Fig. 5, the particle size of the detected fine particle 141 is larger if a peak height of a pulse is greater. Further, the number of peaks of a pulse indicates the number of fine particles 141. Thus, it is possible to determine the particle size of the fine particle 141 detected in the light receiving element 251, on the basis of the intensity of a voltage signal, and to determine the concentration of the fine particle 141 from the number of voltage signals.

As described above, it is possible to obtain information regarding a distribution of the concentration of cigarette smoke in a restaurant (arbitrary place) by acquiring measurement data and position information everywhere in the restaurant.

Here, in the case of a light-receiving-element array two millimeters square that includes light receiving elements provided in a 10×10-array arrangement, a particle size of a fine particle that can be estimated using measurement data using scattered light is about 0.01 µm to 100 µm.

Note that, in general, a fine particle has an irregular shape. Here, in an environmental measurement section using laser diffraction/scattering, the particle size of a measurement-target particle that exhibits the same pattern of diffracted and scattered light as a spherical fine particle having a diameter of c µm (c>0) is c µm regardless of the shape of the measurement-target particle.

For example, a cigarette-smoke particle has a particle size of from about 0.01 µm to 1 µm, a mist particle has a particle size of from about 2 µm to 40 µm, a fog particle has a particle size of from about 40 µm to 400 µm, a pollen has a particle size of from about 9 µm to 90 µm, and yellow sand/a cloud of dust has a particle size of from about 10 µm to 100 µm.

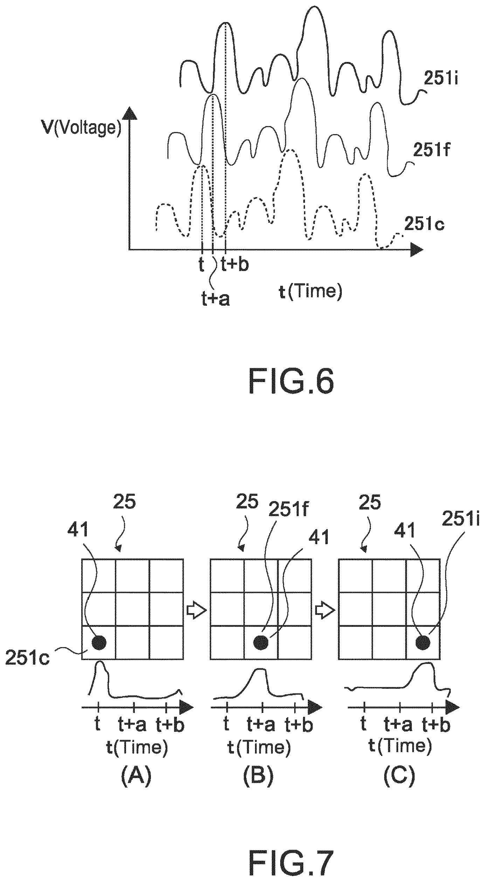

Fig. 6 illustrates an example of pieces of measurement data respectively measured by the light receiving elements 251c, 251f, and 251i of the light-receiving-element array 25 when the fine particle 141 of cigarette smoke moves, for example, from left to right in Fig. 4. In Fig. 6, the pieces of measurement data of the light receiving elements 251f and 251i are displaced upward such that the respective pieces of measurement data of the light receiving elements 251c, 251f, and 251i do not overlap, in order to compare the pieces of measurement data of the light receiving elements 251c, 251f, and 251i. Fig. 7 is a diagram describing detection of a movement of the fine particle 141 of cigarette smoke using the example of pieces of measurement data of Fig. 6.

From comparison of the pieces of measurement data of the respective light receiving elements 251c, 251f, and 251i, it is understood that detected pulses are chronologically shifted, as illustrated in Fig. 6. Further, from comparison of the pieces of measurement data of these light receiving elements, it is understood that the fine particle 141 of cigarette smoke is detected by the light receiving element 251c at a time t, as illustrated in (A) of Fig. 7; the fine particle 141 of cigarette smoke is detected by the light receiving element 251f at a time t+a (a>t), as illustrated in (B) of Fig. 7; and the fine particle 141 of cigarette smoke is detected by the light receiving element 251i at a time t+c (b>a), as illustrated in (C) of Fig. 7. This results in detecting that the fine particle 141 of cigarette smoke moves from the left to the right.

As described above, it is possible to detect the fluidity of cigarette smoke in a restaurant (arbitrary place) by using the light-receiving-element array 25 including a plurality of light receiving elements 251 and by tracking, using measurement data (fine-particle information) detected by each light receiving element 251, how scattered light changes with time when the fine particle 141 passes through laser light. This makes it possible to grasp a temporal change in a distribution of the concentration of cigarette smoke that is information related to cigarette smoke.

As described above, in the environmental measurement section 2 according to the present technology, the use of the light-receiving-element array 25 including a plurality of light receiving elements makes it possible to obtain measurement data of a fine particle of cigarette smoke using each of the plurality of light receiving elements. Thus, there is no need for the air of a certain level of fine-particle concentration for measurement of a fine particle. Further, there is also no need to provide an intake device or the like used to intake air. This makes it possible to make the environmental measurement section 2 small, and thus to install it in a mobile terminal such as a cellular phone. This makes it easy to carry the environmental measurement section 2.

As a result of making it easy to carry an environmental measurement apparatus, as described above, it is possible for an individual to measure a fine particle around the individual.

Thus, it is possible for a large number of unspecified users to have an environmental measurement apparatus including an environmental measurement section in an unspecified place. This results in being able to acquire pieces of measurement data of a fine particle from the environmental measurement apparatuses in various places by a large number of unspecified users moving, without installing the environmental measurement apparatus in a stationary manner. The acquired measurement data is accumulated in the database 80 described later, and this makes it possible to build the database 80.

Further, the use of such a database enables the server 30 to provide a user with fine-particle information (fine-particle information regarding cigarette smoke in the present embodiment) in an arbitrary place selected by the user.

### [Configuration of Cloud Server]

As illustrated in Fig. 2, the server 30 that is an information processing apparatus includes a communication section 31, a controller 32, the database 80, and a storage 81.

The communication section 31 is capable of communicating with the cellular phone 10. The communication section 31 receives, from the cellular phone 10, measurement data measured by the environmental measurement section 2 and position information regarding a place in which the measurement data has been acquired. On the basis of measurement data extracted by the controller 32 referring to the database 80, the communication section 31 transmits, to the cellular phone 10, information related to a fine particle in an arbitrary place, that is, information related to cigarette smoke in a certain restaurant in the present embodiment, in order to provide the information to the user U.

Information for each restaurant is accumulated in the database 80.

Basic information regarding a certain restaurant, position information regarding a position in the restaurant, measurement data of cigarette smoke, time information such as a date and time upon acquirement of the measurement data, and a cigarette-smoke interior map of the restaurant are associated with each other to be accumulated in the database 80, the position information regarding a position in the restaurant and the measurement data of cigarette smoke being associated with each other, the cigarette-smoke interior map of the restaurant being generated on the basis of the accumulated position information and measurement data.

The basic information regarding a restaurant includes position information regarding the restaurant, business-day information, business-hour information, a category and a menu of meals, information regarding the number of tables, position information regarding a position in the restaurant, and the like. The position information regarding a position in the restaurant includes position information regarding each divisional region of a plurality of regions when the restaurant is divided into the plurality of regions.

The cigarette-smoke interior map is information related to a fine particle of cigarette smoke (fine particle in gas) in a restaurant (arbitrary place), and is provided to a user.

A distribution of the concentration of cigarette smoke in the restaurant is reflected in the cigarette-smoke interior map.

The cigarette-smoke interior map includes an entire map in which information regarding cigarette smoke throughout the restaurant is displayed, and a partial map partially extracted from the entire map to be displayed.

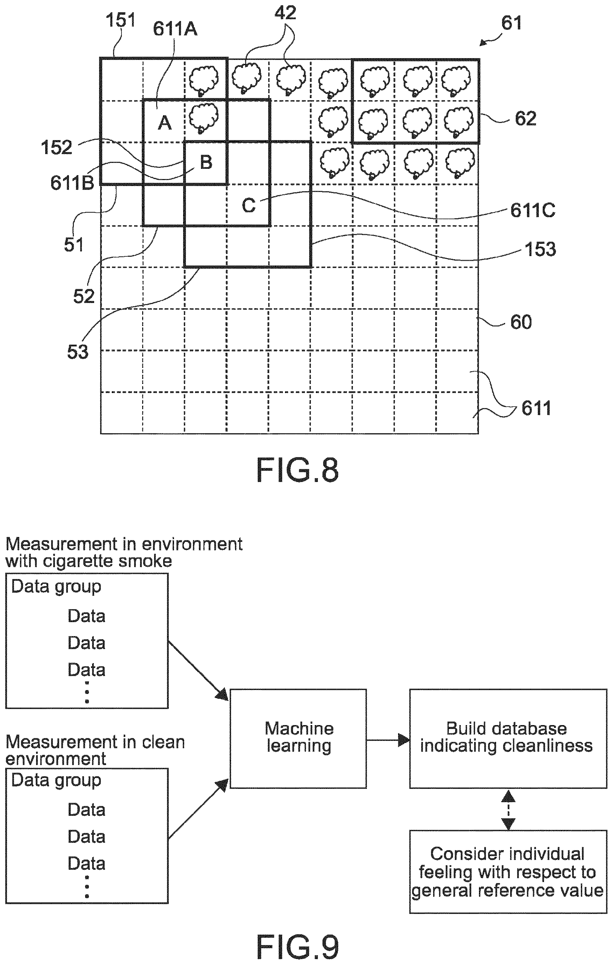

Fig. 8 illustrates an example of an entire map 61. The entire map 61 corresponds to a restaurant 60 as viewed from above. A plurality of divisional regions 611 forms the inside of the restaurant 60. The restaurant 60 is a restaurant that has adopted separation of smoking and nonsmoking areas. In the figure, a smoking area 62 defined by the restaurant 60 is surrounded using a rectangle in a thick solid line. A region other than the smoking area 62 is a nonsmoking area. A smoke mark 42 is given to a position in which it has been determined that a feeling of being bothered by cigarette smoke is caused.

A level of concentration of cigarette smoke that causes a feeling of being bothered by smoke or an odor of a cigarette, differs depending on the individual feeling. However, in the present embodiment, a general value of concentration of cigarette smoke that causes a nonsmoker's feeling of being bothered by smoke or an odor of a cigarette is used as an example of a reference value used to determine whether to give the smoke mark 42. A value of concentration of cigarette smoke that is used for the determination is predetermined.

The entire map 61 illustrated in Fig. 8 indicates that there exists a position, even in a nonsmoking area, in which a feeling of being bothered by smoke or an odor of a cigarette is caused due to the smoke or the odor of a cigarette flowing into the nonsmoking area from the smoking area 62.

Positional information regarding a target divisional region, and each of the pieces of measurement data of cigarette smoke in the target divisional region and in a divisional region surrounding the target divisional region are associated with each other to be chronologically accumulated in the database 80 as a partial map of a cigarette-smoke interior map.

For example, as illustrated in Fig. 8, the position information regarding a position A that is a divisional region 611A, and each of the pieces of measurement data of cigarette smoke in the divisional region 611A and eight divisional regions 611 surrounding the divisional region 611A are associated with each other to be accumulated in the database 80 as a partial map 51 around the position A.

The divisional region 611A and the eight divisional regions 611 surrounding the divisional region 611A are regions surrounded using a rectangle 151 in a thick solid line. The partial map 51 is information regarding cigarette smoke around the position A.

Likewise, the position information regarding a position A (C) that is a divisional region 611B (611C), and each of the pieces of measurement data of cigarette smoke in the divisional region 611B (611C) and eight divisional regions 611 surrounding the divisional region 611B (611C) are associated with each other to be accumulated in the database 80 as a partial map 52 (53) around the position B (C).

The divisional region 611B (611C) and the eight divisional regions 611 surrounding the divisional region 611B (611C) are regions surrounded using a rectangle 152 (153) in a thick solid line. The partial map 52 (53) is information regarding cigarette smoke around the position B (C).

The partial map is generated for each divisional region 611.

As illustrated in Fig. 8, the smoke mark 42 is given to the entire map 61 and a partial map generated on the basis of the entire map 61, and information related to cigarette smoke in a restaurant is provided to the user U by such maps being displayed on the display section 6 of the cellular phone 10.

It is possible for the user U to select a table in which a feeling of being bothered by cigarette smoke is not caused, by referring to a cigarette-smoke interior map.

Training data for measurement data of cigarette smoke that is accumulated in advance by machine learning is accumulated in the database 80. Whether to give the smoke mark 42 to a cigarette-smoke interior map is determined using the training data.

Further, the type of fine particle and measurement data measured by the environmental measurement section may be associated with each other to be accumulated in the database 80. It is possible to estimate the particle size of a fine particle from a pattern of measurement data, and thus to narrow down the type of fine particle to some extent from the particle size of the fine particle to perform estimation.

It is possible to update the database 80 as necessary on the basis of pieces of position information and pieces of measurement data that are acquired from a plurality of cellular phones 10 serving as environmental measurement apparatuses.

The controller 32 includes an acquisition section 33, a calculator 34, a determination section 35, an extraction section 36, an information providing section 37, a database building section 38, a learning section 39, an estimator 40, and a map generator 41.

The acquisition section 33 acquires, from each of the plurality of cellular phones 10,
measurement data of the environmental measurement section 2 and position information regarding a place in which the measurement data has been acquired.

The calculator 34 calculates the concentration distribution, the fluidity, and the like of cigarette smoke in a certain restaurant on the basis of the position information and the measurement data that are acquired by the acquisition section 33.

The determination section 35 determines whether a concentration of cigarette smoke that is estimated by the estimator 40 described later is equal to or greater than a reference value.

On the basis of position information regarding the current location of the user U, the extraction section 36 refers to the database 80, and extracts, for example, measurement data associated with the position information regarding the user U, and information related to cigarette smoke (fine particle).

The map generator 41 generates a cigarette-smoke interior map using a result of the determination performed by the determination section 35 with respect to the measurement data extracted by the extraction section 36. Specifically, as illustrated in Fig. 8, the map generator 41 generates a cigarette-smoke interior map in which the smoke mark 42 is given to the divisional region 611 exhibiting a concentration of cigarette smoke determined to be equal to or greater than a reference value, and the smoke mark 42 is not given to the divisional region 611 exhibiting a concentration of cigarette smoke determined to be less than the reference value. The cigarette-smoke interior map is accumulated in the database 80.

On the basis of the generated entire map 61, the map generator 41 generates a partial map of the cigarette-smoke interior map for each divisional region 611. The generated partial map of the cigarette-smoke interior map is accumulated in the database 80.

The information providing section 37 provides information related to cigarette smoke to the user U.

As information related to cigarette smoke in the restaurant, the information providing section 37 transmits the cigarette-smoke interior map accumulated in the database 80 to the cellular phone 10 through the communication section 31, the cigarette-smoke interior map being extracted on the basis of the position information regarding the user U.

Note that, an entire map may be displayed on the display section 6 of the cellular phone 10 such that it is possible to confirm a state of cigarette smoke throughout a restaurant. Alternatively, a partial map may be displayed on the display section 6 of the cellular phone 10 such that it is possible to enlarge to confirm a state of cigarette smoke around the current location of the user U. It may be possible for the user U to select the display method discretionarily.

Further, the information providing section 37 refers to the cigarette-smoke interior map, and transmits information indicating a favorable behavior of the user U to the cellular phone 10 through the communication section 31, the information indicating a favorable behavior of the user U being determined on the basis of information regarding cigarette smoke in the present position of the user U in the restaurant and around the present position of the user U. For example, when the user U does not like cigarette smoke, the information providing section 37 suggests moving to a place, in the restaurant, in which a feeling of being bothered by cigarette smoke is not caused.

As described above, it is possible for the user U to determine the destination and act while avoiding cigarette smoke, by receiving information related to cigarette smoke such as a cigarette-smoke interior map.

The database building section 38 builds the database 80 by chronologically accumulating, in the database 80, time information, measurement data of a fine particle, and position information in association with each other, the measurement data of a fine particle and the position information being acquired by the acquisition section 33.

Further, the database building section 38 builds the database 80 using a result of learning performed by the learning section 39.

Furthermore, the database building section 38 checks, against the database 80, the associated position information and measurement data that are acquired by the acquisition section 33, determines, using an arbitrary algorithm, whether to update the database 80, and constructs constantly up-to-date data.

Moreover, the database building section 38 builds the database 80 by chronologically accumulating a cigarette-smoke interior map generated by the map generator 41 in the database 80 for each restaurant.

The learning section 39 performs machine learning using position information and measurement data that are acquired from the environmental measurement section 2 installed in each of the plurality of cellular phones 10, performs statistical processing with respect to a relationship between the position information and the measurement data, and extracts correlation information regarding a correlation between the position information and the measurement data.

Further, in an initial stage of building of the database 80, the learning section 39 performs machine learning using measurement data acquired in a clean environment without cigarette smoke and measurement data acquired in an environment with cigarette smoke. The measurement data of cigarette smoke is accumulated in the database 80 as training data.

Note that, in the present embodiment, an example in which whether to give the smoke mark 42 to a cigarette-smoke interior map is determined using a general reference value, is described. However, an individual feeling of the user U who uses the cigarette-smoke interior map may be considered. This will be described in a sixth embodiment.

The estimator 40 refers to the training data accumulated in the database 80 to estimate whether the measurement data measured by the environmental measurement section 2 is measurement data of cigarette smoke. Further, the estimator 40 refers to the database 80 to estimate the concentration of cigarette smoke from the measurement data measured by the environmental measurement section 2.

The storage 81 includes a memory device such as a RAM, and a nonvolatile recording medium such as a hard disk drive, and stores therein a program used to cause the server 30 to perform processing related to provision of fine-particle information.

The program stored in the storage 81 is used to cause the server 30 that is an information processing apparatus to perform a process including acquiring measurement data and position information regarding a place in which the measurement data has been acquired, the measurement data and the position information being acquired by each of the plurality of environmental measurement sections; and performing statistical processing with respect to a relationship between the position information and the measurement data that are acquired from each of the plurality of environmental measurement sections.

### [Database Building Method]

It is possible to build the database 80 by actively creating a clean environment without cigarette smoke and an environment with cigarette smoke to experimentally perform machine learning in advance. Fig. 9 illustrates an example of building of the database 80.

As illustrated in Fig. 9, in a clean environment without cigarette smoke and in an environment with cigarette smoke, measurement of a fine particle is performed using the environmental measurement section 2 to acquire measurement data.

Next, machine learning is performed using a data group including a plurality of pieces of measurement data acquired in the clean environment and using a data group including a plurality of piece of measurement data acquired in the environment with cigarette smoke. Accordingly, it is possible to build the database 80 indicating the cleanliness of air.

The cleanliness of air is related to the concentration of cigarette smoke. A reference value used to determine whether a feeling of being bothered by cigarette smoke is caused is related to the concentration of cigarette smoke. The measurement data includes information regarding the concentration of cigarette smoke. The measurement data and the concentration of cigarette smoke being estimated from the measurement data are associated with each other to be accumulated in the database 80.

Note that, in the present embodiment, an example in which whether to display the smoke mark 42 on a cigarette-smoke interior map is determined using a general reference value, is described. However, an individual feeling of the user U who uses the cigarette-smoke interior map may be considered. This will be described in a second embodiment described later.

Such an experiment results in a plurality of pieces of measurement data of cigarette smoke being accumulated in the database 80, and the measurement data acquired in advance is used as training data.

Then, measurement data actually measured by the environmental measurement section 2 is checked against the training data, and this makes it possible to determine whether the actually measured measurement data is measurement data of cigarette smoke. Further, it is possible to determine whether the concentration of cigarette smoke being indicated by the actually measured measurement data is equal to or greater than a reference value.

Pieces of fine-particle information and pieces of position information that are obtained from a large number of unspecified people who have an environmental measurement apparatus including the environmental measurement section 2, are accumulated as necessary in the database 80 built by performing such an experiment, and the database 80 is updated. This also enables the user U to acquire up-to-date cigarette-smoke interior information and to grasp cigarette-smoke information in real time.

Further, information is chronologically built in the database 80. Thus, it is possible to acquire a cigarette-smoke interior map of the same restaurant for each period of time. For example, the proportion of smokers to customers of a restaurant may be changed according to the period of time even in the case of the same restaurant. In this case, the cigarette-smoke interior map differs depending on the period of time.

The above-described chronological building of information enables the user U to select the period of time to visit a restaurant by referring to and comparing maps of respective periods of time.

[Information Processing Method Performed in Information Processing System] Next, a specific information processing method performed in the information processing system 1 is described with reference to Figs. 8, 10, and 11.

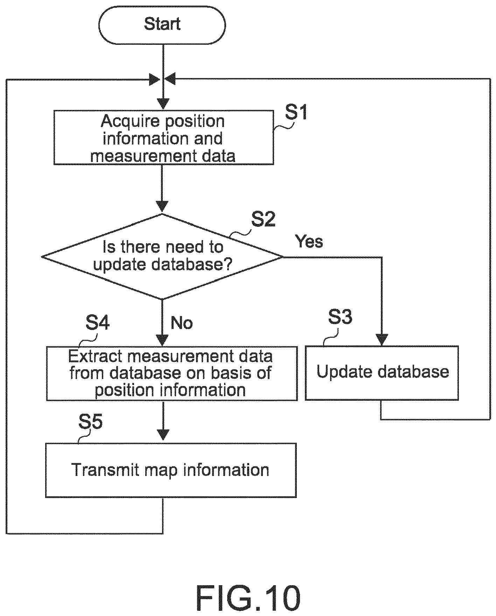

Fig. 10 is a flowchart describing an information processing method related to provision of cigarette-smoke information (provision of fine-particle information) that is performed in the server 30.

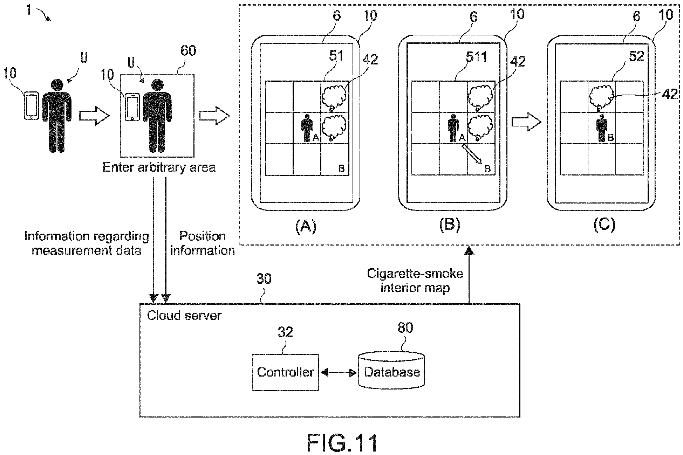

Fig. 11 schematically illustrates how the user U starts an application and uses a cigarette-smoke interior map in the information processing system 1.

Here, an example in which the user U enters the restaurant 60 that is an arbitrary place, and obtains a cigarette-smoke interior map of the restaurant 60, is described.

As illustrated in Fig. 11, the user U starts an application and enters the restaurant 60 that is an arbitrary place. Due to the application being started, measurement of cigarette smoke is performed by the environmental measurement section 2 to acquire measurement data. Further, position information regarding a place in which the measurement data has been acquired is acquired by the GPS 7. The position information and the measurement data are transmitted to the server 30.

As illustrated in Fig. 10, the server 30 acquires the position information and the measurement data from the cellular phone 10 (S1). Here, it is assumed that position information regarding a position A is acquired as the position information.

Next, the server 30 refers to measurement data associated with the acquired position information using the database 80, and determines whether to update the database 80 (S2).

Whether to update the database 80 is determined according to, for example, whether measurement data associated with the position information is sufficiently accumulated, or whether the acquired measurement data is analog to measurement data accumulated in the database 80.

When it has been determined that the update is to be performed, the process moves onto S3. The position information and the measurement data that are acquired in S1 are accumulated in the database 80 in association with time information, and the database 80 is updated (S3).

When it has been determined that the update is not to be performed, the process moves onto S4.

Using the database 80, the server 30 extracts measurement data and a cigarette-smoke interior map that are associated with the position information acquired in S1 (S4).

Next, the controller 32 transmits, to the cellular phone 10, the extracted partial map 51 around the position A, the extracted partial map 51 being given the smoke mark 42 (S5). Further, a notification regarding the change of a table may be performed together with the transmission of the partial map.

When the cellular phone 10 receives the partial map 51, the cellular phone 10 displays the partial map 51 on the display section 6 as illustrated in (A) of Fig. 11. For example, it is possible for the user U to consider moving to the position B without the smoke mark 42, while viewing the partial map 51. The current location of the user U is displayed on the partial map 51 using a human-shaped mark.

Further, when the notification regarding the change of a table is performed, an image such as a partial map 511 illustrated in (B) of Fig. 11 is displayed, the image being an image in which a suggestion that the user U move from the present position A to the position B is indicated using an arrow. Alternatively, a notification of "it is recommended that you move to another table" may be performed by displaying a text or by sound to suggest that the user U move to another table. On the other hand, when there is no cigarette smoke around the user U, a notification of "there is no need to move to another table" is performed by displaying a text or by sound.

After S5, when, for example, the user U moves from the position A to the position B and there is a change in position information regarding the user U, the process returns to S1, and the processes of S1 to S5 are repeatedly performed. When the user U moves to the position B, the partial map 52 including information regarding cigarette smoke in the position B and around the position B is displayed on the display section 6, as illustrated in (C) of Fig. 11.

As described above, after the user U moves, information is accumulated in the database 80 by the processes of S1 to S5 being repeatedly performed. This results in improving the accuracy in provided fine-particle information and in improving convenience.

Further, when, for example, there exists a plurality of users U each having an environmental measurement apparatus at the same time in the same restaurant, it is possible to acquire information regarding cigarette smoke in the restaurant in real time. This enables the user U to acquire an up-to-date cigarette-smoke interior map.

Note that, in the present embodiment, the example in which the user U directly goes to a restaurant has been described, but the present disclosure is not limited to this example. When the user U wants to know, in advance, information regarding cigarette smoke in a restaurant without going to the restaurant, it is also possible to obtain the information regarding cigarette smoke in the restaurant.

In this case, the user U who is provided with information regarding cigarette smoke in a restaurant does not necessarily have to have an environmental measurement apparatus. It is sufficient if the user U has a terminal that is capable of communicating with the server 30 that provides the cigarette-smoke information.

Information regarding cigarette smoke in a target restaurant is provided to the terminal of the user U from the server 30 by transmitting, from the terminal of the user U to the server 30, information with which the address of the restaurant can be specified, such as the name of the restaurant.

As described above, it is possible to make the environmental measurement section 2 according to the present embodiment small. Thus, for example, the environmental measurement section 2 can be carried by being installed in a cellular phone or the like, or the environmental measurement section 2 can be carried in the form of a card environmental measurement apparatus. This enables a large number of unspecified people to have an environmental measurement apparatus. The environmental measurement apparatuses carried by the large number of unspecified people enable the server 30 to acquire a plurality of pieces of measurement data of cigarette smoke in various places, and thus to acquire information regarding cigarette smoke in all of the places over a wide range.

Note that, in the present embodiment, the example in which the user U who goes to a restaurant uses a cigarette-smoke interior map has been described, but a restaurant may also use a cigarette-smoke interior map. In order to prevent smoke, odor, and the like of a cigarette from flowing into a nonsmoking area from a smoking area, a restaurant may also make use of a cigarette-smoke interior map when the restaurant considers where to install emission equipment or when the restaurant considers moving a smoking area.

Modifications of the environmental measurement section are described below as the second embodiment to a fifth embodiment. Structural elements similar to those in the embodiment described above are denoted by similar reference symbols, and descriptions thereof may be omitted. Further, only the structure of the environmental measurement section is described.

### (Second Embodiment)

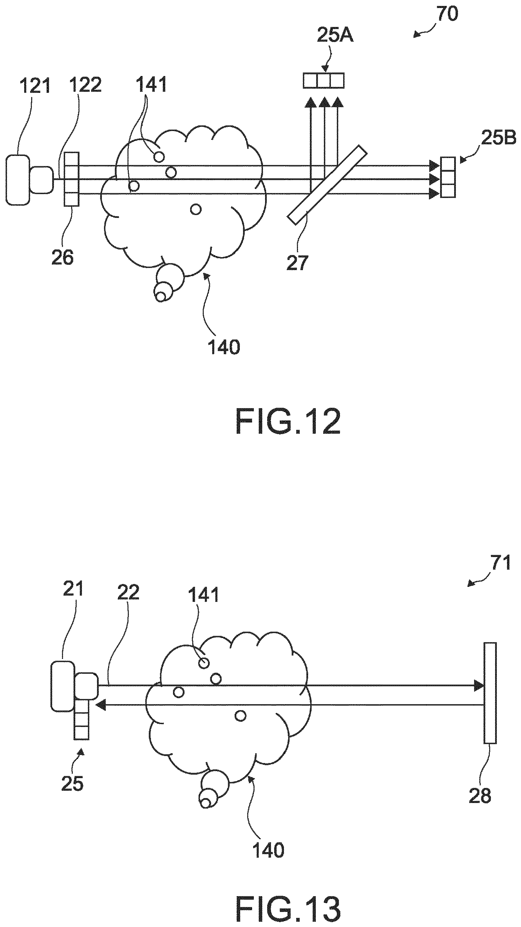

Fig. 12 schematically illustrates an environmental measurement section according to the present embodiment.

As illustrated in Fig. 12, an environmental measurement section 70 includes a laser source 121, two light-receiving-element arrays 25A and 25B, a dichroic mirror 27, and a diffraction grating 26. The light-receiving-element arrays 25A and 25B each have the same configuration as the light-receiving-element array 25 described above.

The laser source 121 includes a plurality of laser sources each emitting laser light of a different wavelength. The laser source 121 is capable of simultaneously or selectively emitting pieces of laser light of a plurality of different wavelengths. Here, it is assumed that the laser source 121 emits laser light 122 including pieces of laser light of a plurality of different wavelengths.

The diffraction grating 26 disperses the laser light 122 from the laser source 121.

From among pieces of light obtained by the dispersion performed by the diffraction grating 26, a piece of light of a certain wavelength is reflected off the dichroic mirror 27 to enter the light-receiving-element array 25A, and a piece of light of a wavelength other than the certain wavelength is transmitted through the dichroic mirror 27 to enter the light-receiving-element array 25B.

With respect to backscattered light generated by the fine particle 141 in the gas 140 such as cigarette smoke being irradiated with light obtained by dispersion performed by the diffraction grating 26, the dichroic mirror 27 causes light of a certain wavelength to enter the light-receiving-element array 25A and causes light of a wavelength other than the certain wavelength to enter the light-receiving-element array 25B in the environmental measurement section 70. Wavelengths of pieces of light that respectively enter the light-receiving-element array 25A and the light-receiving-element array 25B are different from each other, and pieces of measurement data measured by the respective light-receiving-element arrays are different from each other.

As described above, the environmental measurement section 70 of the present embodiment makes it possible to obtain a measurement pattern of a measurement-target fine particle for each wavelength of different laser light irradiated onto the fine particle. This makes it possible to more finely classify the type of measurement-target fine particle using measurement data measured by the environmental measurement section 70, and this results in improving the accuracy in estimating the type of fine particle.

### (Third Embodiment)

Fig. 13 schematically illustrates an environmental measurement section according to the present embodiment.

As illustrated in Fig. 13, an environmental measurement section 71 may include the laser source 21, the light-receiving-element array 25, and a total reflection mirror 28.

In the present embodiment, the light-receiving-element array 25 is arranged close to the laser source 21. Further, as a result of providing the total reflection mirror 28, backscattered light generated by the fine particle 141 in the gas 140 being irradiated with the laser light 22 emitted from the laser source 21, is totally reflected off the total reflection mirror 28 to enter the light-receiving-element array 25.

### (Fourth Embodiment)

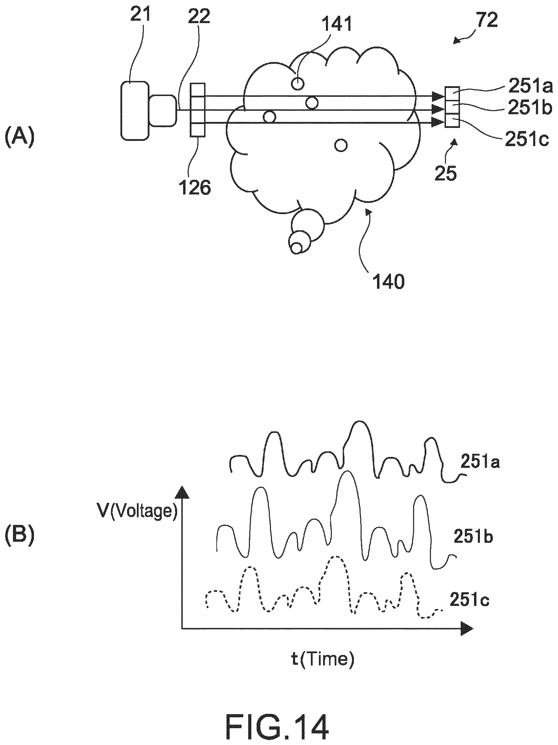

(A) of Fig. 14 schematically illustrates an environmental measurement section according to the present embodiment.

As illustrated in (A) of Fig. 14, an environmental measurement section 72 includes the laser source 21, the light-receiving-element array 25, and a diffraction grating 126.

The diffraction grating 126 divides the laser light 22 from the laser source 21 into pieces of laser light of, for example, 0th-order light, +/-first-order light, and +/-second-order light. Backscattered light generated by the laser light obtained by the division being irradiated onto the fine particle 141 in the gas 140 such as cigarette smoke, enters the light-receiving-element array 25.

(B) of Fig. 14 illustrates an example of pieces of measurement data of the light receiving elements 251a, 251b, and 251c of the light-receiving-element array 25. In (B) of Fig. 14, the pieces of measurement data of the light receiving elements 251b and 251c are displaced upward such that the respective pieces of measurement data of the light receiving elements 251a, 251b, and 251c do not overlap.

In the example illustrated in (B) of Fig. 4, the light receiving element 251b receives the first-order light, and the light receiving elements 261a and 251c receive the second-order light of the same scattered light as the first-order light. As illustrated in the figure, the intensities of the first-order light and the second-order light are different even in the case of the same scattered light, and their measured measurement patterns are different. Thus, it is possible to more accurately detect where a fine particle is situated using the measurement patterns, and to more accurately detect a distribution of fine particles and a change in the distribution. Further, the accuracy in estimating the type of fine particle is improved.

### (Fifth Embodiment)

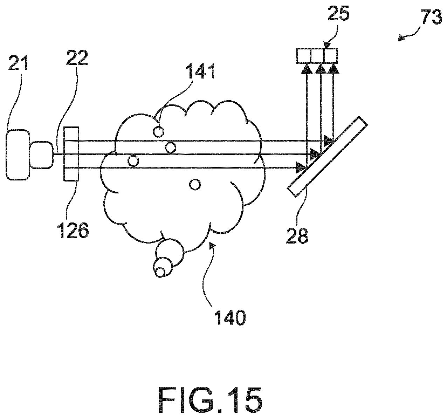

Fig. 15 schematically illustrates an environmental measurement section according to the present embodiment.

As illustrated in Fig. 15, an environmental measurement section 73 includes the laser source 21, the light-receiving-element array 25, the diffraction grating 126, and the total reflection mirror 28.

The present embodiment is different from the fourth embodiment only in that the total reflection mirror 28 is further included and light that is totally reflected off the total reflection mirror 28 enters the light-receiving-element array 25.

The above-described inclusion of the total reflection mirror 28 makes it possible to broaden a range for designing the arrangement of the light-receiving-element array 25, and to change the arrangement of the light-receiving-element array 25 discretionarily depending on an apparatus in which the environmental measurement section 73 is installed.

### (Sixth Embodiment)

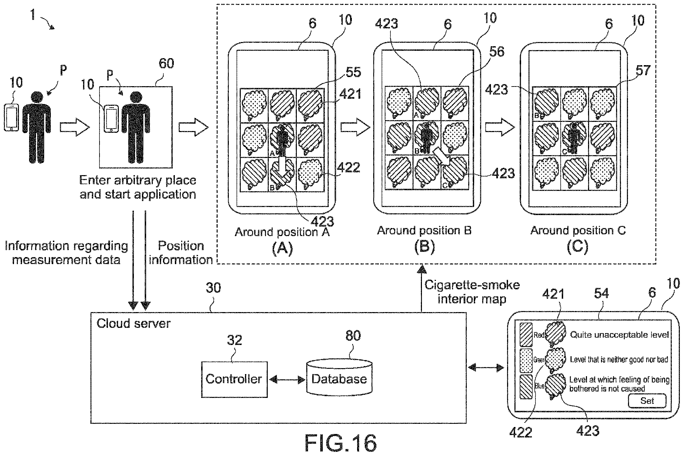

In the first embodiment, whether to give a smoke mark to a cigarette-smoke interior map is determined by whether a concentration of cigarette smoke that is indicated by measurement data is equal to or greater than a predetermined reference value. However, a smoke mark may be given in consideration of an individual feeling of the user U who uses a cigarette-smoke interior map. The description is made below using Fig. 16. Note that structural elements similar to those in the embodiments described above are denoted by similar reference symbols, and descriptions thereof may be omitted. Here, the description is made focused on a point different from that of the first embodiment.

Fig. 16 schematically illustrates how the user U starts an application and uses a cigarette-smoke interior map in the information processing system 1. Further, the controller 32 illustrated in Fig. 16 has the same configuration as the controller 32 of the first embodiment. Although an illustration of a detailed functional block diagram of the controller 32 is omitted in Fig. 16, the description is made using the configuration of the functional block illustrated in Fig. 2 as necessary.

The acquisition section 33 acquires information regarding a feeling of the user U in addition to position information and measurement data. The information regarding a feeling is information related to an individual feeling of the user U with respect to cigarette smoke. The acceptable level with respect to smoke and an odor of a cigarette differs from individual to individual, and, in the present embodiment, a cigarette-smoke interior map is generated in consideration of an individual feeling of the user U.

The information regarding a feeling of the user U is set by an input operation performed by the user U from a self-level setting screen 54 displayed on the display section 6 of the cellular phone 10, for example, as illustrated in Fig. 16. The self-level setting screen 54 allows the user U to select, in the current location of the user U, the level of a feeling with respect to cigarette smoke from three levels that are a "quite unacceptable level", a "level that is neither good nor bad", and a "level at which a feeling of being bothered is not caused".

When the level is selected by the user U, the acquisition section 33 acquires measurement data of the environmental measurement section 2 and information regarding a selected level that is information regarding a feeling of the user U.

When information including the selected level and the measurement data that are associated with each other is acquired multiple times, the learning section 39 performs machine learning using these pieces of information, and extracts information regarding a correlation between measurement data and a selected level.

On the basis of the information regarding a correlation between a selected level and measurement data that is extracted by the learning section 39, the determination section 35 determines which of the levels the concentration of cigarette smoke that is indicated by the measurement data acquired by the environmental measurement section 2 corresponds to for the user U.

The map generator 41 generates a cigarette-smoke interior map using a result of the determination performed by the determination section 35. For each divisional region 611, one of color-coded smoke marks 421 to 423 respectively corresponding to the three levels is given to the cigarette-smoke interior map of the present embodiment. In Fig. 16, red is shown using upward-sloping lines, green is shown using dots, and blue is shown using downward-sloping lines.

The information providing section 37 provides the user U with a map in which information regarding a feeling of the user has been considered.

As illustrated in Fig. 16, a cigarette-smoke interior map is generated, the cigarette-smoke interior map being a cigarette-smoke interior map in which the red smoke mark 421 is given to a divisional region of the "quite unacceptable level", the green smoke mark 422 is given to a divisional region of the "level that is neither good nor bad", and the blue smoke mark 423 is given to a divisional region of the "level at which a feeling of being bothered is not caused".

The cigarette-smoke interior map is transmitted to the cellular phone 10. A partial map centered at the location of the user U is displayed on the display section 6 of the cellular phone 10, the partial map providing information regarding cigarette smoke around the location of the user U.

(A) to (C) of Fig. 16 are plan views of the cellular phone 10, and are diagrams for describing images displayed on the display section 6.

In (A), a partial map 55 around the position A in the restaurant (arbitrary place) 60 is displayed. In (B), a partial map 56 around the position B in the restaurant is displayed. In (C), a partial map 57 around the position C in the restaurant is displayed.

An arrow suggesting a direction of a moving destination may be displayed on a partial map, as illustrated in (A) and (B). It is possible for the user U to select a table in which a feeling of being bothered by cigarette smoke is not caused, by moving to a position on which the blue smoke mark 423 is displayed while viewing an image displayed on the display section 6.

Information regarding cigarette smoke in which information regarding a feeling of the user U has been considered may be acquired, as described above.

### (Seventh Embodiment)

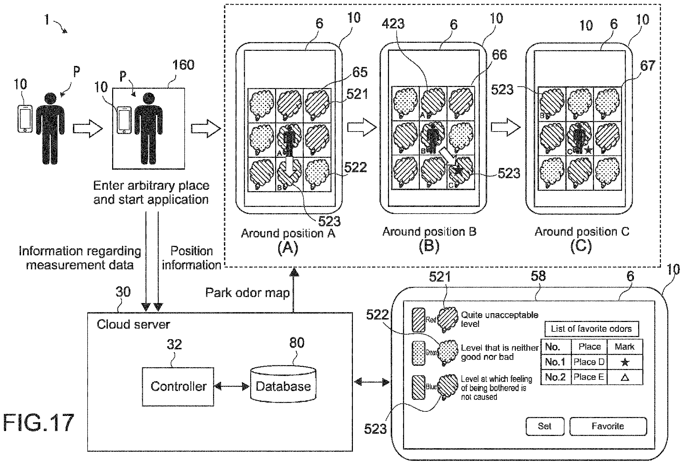

In the embodiments described above, the example of measuring a fine particle of cigarette smoke using an environmental measurement section has been described. However, the measurement-target fine particle is not limited to this. For example, a favorite odor of the user U may be registered to build the database 80. The description is made below using Fig. 17. Note that structural elements similar to those in the embodiments described above are denoted by similar reference symbols, and descriptions thereof may be omitted.

Fig. 17 schematically illustrates how the user U starts an application and uses an odor map of an arbitrary place in the information processing system 1. It is possible to indicate, on the map, a place in which there exists a favorite odor of the user U. In the present embodiment, an example in which an arbitrary place is a park 160 is described.

Position information regarding a park, position information regarding a position in the park, measurement data regarding an odor, the level of odor, and time information such as a date and time upon acquirement of the measurement data are associated with each other to be accumulated in the database 80, the position information regarding a position in the park, the measurement data regarding an odor, and the level of odor being associated with each other. The position information regarding a position in the park includes position information regarding a position in each divisional region of a plurality of regions when the park is divided into the plurality of regions. The level of odor is information regarding a feeling of the user that is determined by the user.

Further, a map of a park odor in the park 160 that is generated on the basis of the position information and measurement data associated with each other is chronologically accumulated in the database 80. The park odor map is information related to a fine particle of an odor (fine particle in gas) in a park (arbitrary place), and is provided to a user.

The park odor map includes an entire map that indicates a distribution of odors throughout the park, and a partial map partially extracted from the entire map.

For each divisional region, one of color-coded smoke marks 521 to 523 respectively corresponding to levels for an odor is given to the park odor map. With respect to a level for an odor that is information regarding a feeling of the user U, there exist three levels that are a "quite unacceptable level" (the red smoke mark 521), a "level that is neither good nor bad" (the green smoke mark 522), and a "level at which a feeling of being bothered is not caused" (the blue smoke mark 523). Which of the levels is to be given is determined by statistical processing being performed using the server 30 with respect to a result of the determinations regarding an odor that are performed by a plurality of users. The determination regarding an odor is performed by each user U from a self-level setting screen 58 illustrated in Fig. 17.

Information related to an odor in a certain park is accumulated, the information being obtained from a large number of unspecified of people. This results in extracting correlation information indicating that "something" exhibiting measurement data exists in a place H situated in the park when the origin of the odor is unknown, but when, for example, pieces of measurement data that are measured in the place H and obtained from a plurality of people are the same. The measurement data specifies "something" that is the origin of the odor.

Further, statistical processing is performed on a level selected by each user U with respect to the odor of "something". This results in extracting correlation information indicating that the odor of "something" in the place H situated in the park is an odor at the "level at which a feeling of being bothered is not caused", and in building the database 80. Then, on the basis of the correlation information, it is possible to generate a park odor map to which a level of odor that is information regarding a feeling is given. The correlation information is extracted by machine learning being performed by the learning section 39.

Note that, in the first embodiment described above, the example in which a value of concentration of cigarette smoke that is a reference value used to determine whether to give the smoke mark 42 is predetermined, has been described. However, as in the case of the present embodiment, statistical processing may be performed by machine learning using pieces of measurement data of cigarette smoke that are collected from a large number of unspecified people and using the level of a feeling of a user with respect to smoke upon acquiring the pieces of measurement data, information regarding a correlation between measurement data and a level of a feeling that is information regarding a feeling may be extracted, and whether to give the smoke mark 42 may be determined on the basis of the extracted correlation information.

Further, in the present embodiment, when there exists an odor that the user U likes, it is possible to favorite, from the self-level setting screen 58, a place in which there exists the odor and measurement data.

In addition to a selection button used to perform the above-described determination of a level of odor, a button used to favorite an odor, and a list of odors marked as favorites are displayed on the self-level setting screen 58.

When the user U selects a button "favorite" on the self-level setting screen 58, measurement data of the odor, position information, time information, and information indicating that the odor is a favorite odor are associated with each other to be accumulated in the database 80, the information indicating that the odor is a favorite odor being information regarding a feeling of the user U. When the user U favorites an odor, a list of favorite odors is displayed on the self-level setting screen 58.

For example, in the example of Fig. 17, a place (here, a place D) upon favoriting an odor and a mark (in this case, a star mark) are displayed in a list number 1 of the list of favorite odors, the mark being displayed on a map when measurement data is detected that is the same as the measurement data acquired upon favoriting the odor. Likewise, a place E upon favoriting an odor and a triangle mark are displayed in a list number 2.

The information providing section 37 provides a map to the user U as information related to a fine particle, the map being given a mark indicating a place in which there exists an odor that is a favorite of the user U.

For example, the user U enters an arbitrary place and starts an application to obtain a map of the place. When an odor corresponding to measurement data that is the same as measurement data of an odor previously marked as a favorite is detected in the place and accumulated in the database 80, it is possible to obtain a map to which a mark indicating the odor is given. For example, it is possible to obtain a map to which a star mark is given when it is an odor of the list number 1.

Next, an example of providing odor information (fine-particle information) to the user U using the database 80 built as described above, is described.

When the user U enters a park and starts an application in order to obtain fine-particle information, position information regarding the current location of the user U and measurement data obtained by the environmental measurement section 2 are transmitted to the server 30.

The server 30 transmits, to the cellular phone 10, a map around the user in which a position of the current location of the user U is indicated. The cellular phone 10 displays the map on the display section 6. For example, (A) of Fig. 17 is a partial map 65 of a park odor map that is displayed when the user U is in the position A. (B) is a partial map 66 that is displayed when the user U is in the position B, and (C) is a partial map 67 that is displayed when the user U is in the position C.

The color-coded smoke marks 521 to 523 respectively corresponding to levels for an odor are displayed on the partial maps 65 to 67, and a mark indicating that there exists an odor that is a favorite of the user U is also displayed on the partial maps 65 to 67 when there exists such an odor.

A star mark indicating that there exists an odor of the list number 1 that is a favorite of the user U, is displayed on the position C of the partial maps illustrated in Fig. 17. As illustrated in (A) and (B), an arrow suggesting a moving direction to the user U is displayed on the partial maps such that it is possible for the user U to move efficiently to the position C.

Odor information that is fine-particle information may be provided to the user U, as described above, or a favorite odor may be marked as a favorite.

Note that when the origin of an odor is known to the user U, the configuration may be made such that the user U can input to favorite a name specifying the origin of the odor, such as a flower, a forest, a grilled meat, and a cigarette. Alternatively, several selection items such as an odor of flower, an odor of forest, an odor of aroma, an odor of cigarette, an odor of food, and other odors may be provided in advance,
and an item that seems appropriate may be selected by user U.

### (Eighth Embodiment)

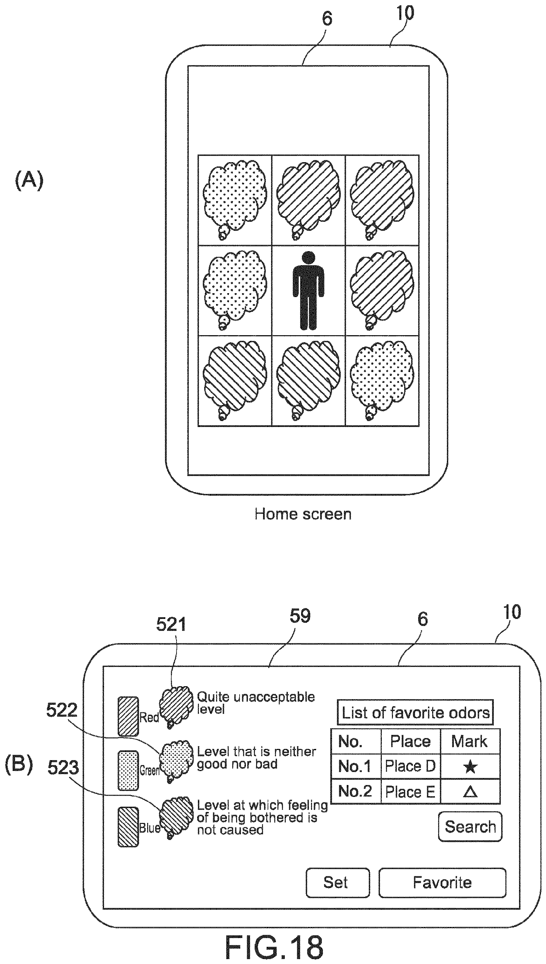

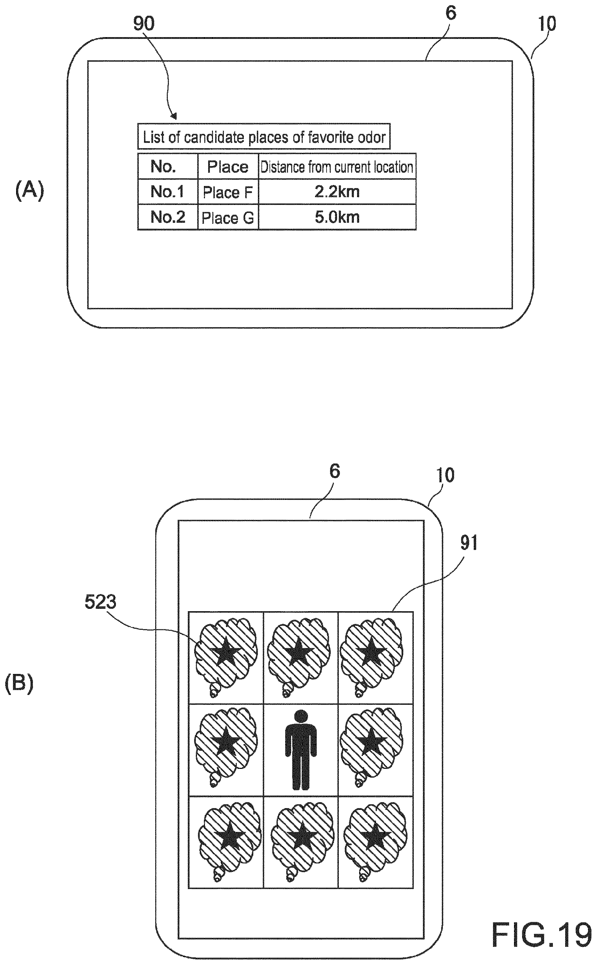

The present embodiment is described using Figs. 18 and 19. Figs. 18 and 19 illustrate examples of images displayed on the cellular phone 10 by information processing being performed in order to provide fine-particle information. In the present embodiment, it is possible to favorite an odor, as in the case of the seventh embodiment. In addition, the server 30 is configured to provide information regarding a place in which there exists a favorite odor marked as a favorite. Structural elements similar to those in the seventh embodiment are denoted by similar reference symbols below, and descriptions thereof may be omitted.

When an application is started by the user U, a home screen is displayed on the display section 6 of the cellular phone 10, as illustrated in (A) of Fig. 18.

Next, an image displayed on the display section 6 is changed to a self-level setting screen 59, as illustrated in (B) of Fig. 18. The self-level setting screen 59 is a screen in which a search button used to search for a place of a favorite odor has been added to the self-level setting screen 59 described in the seventh embodiment. In the present embodiment, the database 80 is built similarly to the seventh embodiment.

In the present embodiment, when the user U selects a favorite odor from the favorite list and presses the search button, a list 90 of candidate places of a favorite odor is displayed on the display section 6, as illustrated in (A) of Fig. 19. A place in which the selected odor has been detected is given in the list 90 of candidate places of a favorite odor.

The list 90 of candidate places of a favorite odor is generated by the server 30 referring to the database 80 and extracting position information associated with measurement data that is the same as measurement data of an odor favorited as a favorite of the user U.

The information providing section 37 provides the list 90 of candidate places of a favorite odor to the user U as information related to a fine particle.

When a candidate place is selected by the user U from the list 90 of candidate places of a favorite odor, an odor map 91 of the candidate place is displayed on the display section 6. As described above, it is possible for the server 30 to provide the user U with information regarding a place in which there exists a favorite odor.

The embodiment of the present technology is not limited to the embodiments described above, and various modifications may be made without departing from the scope of the present technology.

For example, the example in which a cellular phone serves as an environmental measurement apparatus has been described in the embodiments described above. However, the form of the environmental measurement apparatus is not limited to this. For example, the environmental measurement apparatus may be a wearable apparatus such as eyeglasses including an environment measuring section, or an apparatus in the form of, for example, a bracelet or a necklace; or an apparatus in the form of a card.

The environmental measurement apparatus sufficiently small to be portable as described above enables an individual user to measure a fine particle around the individual user. Accordingly, it is possible for an information processing apparatus (the server 30 in the embodiments described above) to accumulate, in a database, pieces of measurement data of a fine particle measured by the environmental measurement apparatuses of a large number of unspecified people in unspecified places, and to provide, using the database, a user with fine-particle information regarding a fine particle in an arbitrary place selected by the user.

It is sufficient if the environmental measurement apparatus includes an environmental measurement section, a position-information acquisition section such as a GPS, and the communication section 3 capable of communicating with an information processing apparatus performing information processing in order to provide fine-particle information.

Further, in the embodiments described above, the cellular phone 10 serves as an environmental measurement apparatus and serves as an apparatus that receives fine-particle information regarding a fine particle in an arbitrary region. However, the environmental measurement apparatus and the apparatus that receives the fine-particle information may be separate apparatuses.

Further, it is possible for a user to move with an environmental measurement apparatus, since it is possible to make the environmental measurement apparatus small. However, the environmental measurement apparatus may be a stationary apparatus or an installation-type apparatus, and fine-particle information may be obtained from both a stationary environmental measurement apparatus and a movable environmental measurement apparatus.

Further, a place in which a fine particle is measured is not limited to a restaurant or a park. It is possible to acquire fine-particle information regarding a fine particle in any places such as a theme park, a station yard, a region around a station, and a school.

Further, although the example in which the information processing apparatus is a cloud server has been described, the controller 32 may be installed in, for example, a cellular phone in which the environmental measurement section 2 is installed. In this case, the cellular phone serves as an environmental measurement apparatus and an information processing apparatus. Further, in this case, the database 80 may be situated in a cloud server that is capable of communicating with the cellular phone 10.

Furthermore, in the embodiments described above, a fine particle of cigarette smoke and a fine particle of an odor have been described as examples of a fine particle in gas, but the fine particle is not limited to them. It is sufficient if the fine particle can be measured using the environment measuring section. The fine particle may be a fog, snow, yellow sand, a cloud of dust, a pollen, or the like.

Note that the present technology may also take the following configurations.
(1) An environmental measurement apparatus, including:
   a light source that irradiates light onto a fine particle in gas; and
   a light-receiving-element array that includes a plurality of light receiving elements receiving backscattered light obtained by the light being irradiated onto the fine particle.
(2) The environmental measurement apparatus according to (1), further including:
   a position-information acquisition section that acquires position information regarding a position of the environmental measurement apparatus; and
   a communication section that communicates another apparatus, and transmits, to the other apparatus, measurement data measured by the light receiving element and the position information.
(3) The environmental measurement apparatus according to (1) or (2), further including
   a diffraction grating that the light from the light source enters, the diffraction grating emitting the light that is to be irradiated onto the fine particle.
(4) The environmental measurement apparatus according to any one of (1) to (3), further including a mirror off which the backscattered light is reflected to enter the light-receiving-element array.
(5) The environmental measurement apparatus according to any one of (1) to (3), further including:
   a plurality of the light-receiving-element arrays; and
   a mirror through which light of a first wavelength from among the backscattered light is transmitted to enter one of the plurality of the light-receiving-element arrays, the mirror being a mirror off which light of a second wavelength that is different from the first wavelength is reflected to enter another of the plurality of the light-receiving-element arrays.
(6) An information processing apparatus, including
   an acquisition section that acquires measurement data of a fine particle in gas, and position information regarding a place in which the measurement data has been acquired, the measurement data and the position information being acquired by an environmental measurement section that includes a light source and a light-receiving-element array, the light source emitting light, the light-receiving-element array including a plurality of light receiving elements receiving backscattered light obtained by the emitted light being irradiated onto the fine particle.
(7) The information processing apparatus according to (6), in which
   the acquisition section acquires the pieces of measurement data and the pieces of position information, the pieces of measurement data being respectively acquired by a plurality of the environmental measurement sections, the pieces of position information being respectively acquired by the plurality of the environmental measurement sections.
(8) The information processing apparatus according to (7), further including
   a learning section that performs statistical processing with respect to a relationship between the position information and the measurement data that are acquired from each of the plurality of the environmental measurement sections.
(9) The information processing apparatus according to any one of (6) to (8), in which
   the acquisition section acquires the measurement data of the fine particle and feeling information regarding a feeling of a user with respect to the fine particle upon acquiring the measurement data, and
   the learning section performs statistical processing with respect to a relationship between the measurement data obtained from each of the plurality of the environmental measurement sections, and the feeling information.
(10) The information processing apparatus according to any one of (6) to (9), further including
   a database building section that builds a database by accumulating therein the measurement data and position information regarding a region in which the measurement data has been acquired, the measurement data and the position information regarding the region being accumulated in a state of being associated with each other, the measurement data and the position information regarding the region being acquired by the environmental measurement section.
(11) The information processing apparatus according to (10), in which
   the database building section accumulates therein information related to the fine particle, the measurement data, and the position information regarding the region in association with each other, the information related to the fine particle being generated on the basis of the measurement data and the position information regarding the region, and
   the information processing apparatus further includes an extraction section that refers to the database and extracts information related to a fine particle in gas in an arbitrary place selected by the user, the information related to the fine particle in the gas in the arbitrary place being associated with position information regarding the arbitrary place.
(12) The information processing apparatus according to (11), further including
   an information providing section that provides the information related to the fine particle in the gas in the arbitrary place, the information related to the fine particle in the gas in the arbitrary place being extracted by the extraction section.
(13) The information processing apparatus according to (12), in which
   the information providing section provides the user with the information related to the fine particle in the gas in the arbitrary place, the information related to the fine particle in the gas in the arbitrary place being information in which the feeling information has been considered.
(14) An information processing system, including:
   a plurality of environmental measurement apparatuses each including a light source and a light-receiving-element array, the light source irradiating light onto a fine particle in gas, the light-receiving-element array including a plurality of light receiving elements receiving the light irradiated onto the fine particle; and
   an acquisition section that acquires measurement data of the fine particle and position information regarding a place in which the measurement data has been acquired, the measurement data and the position information being acquired by each of the plurality of environmental measurement apparatuses.
(15) An information processing method, including
   acquiring a piece of measurement data of a fine particle in gas and a piece of position information regarding a place in which the piece of measurement data has been acquired, the pieces of measurement data and the pieces of position information being acquired by a plurality of environmental measurement sections each including a light source and a light-receiving-element array, the light source irradiating light onto a fine particle in gas, the light-receiving-element array including a plurality of light receiving elements receiving the light irradiated onto the fine particle.
(16) A program that causes an information processing apparatus to perform a process including
   acquiring a piece of measurement data and a piece of position information regarding a place in which the piece of measurement data has been acquired, the pieces of measurement data and the pieces of position information being acquired by a plurality of environmental measurement sections each including a light source and a light-receiving-element array, the light source irradiating light onto a fine particle in gas, the light-receiving-element array including a plurality of light receiving elements receiving the light irradiated onto the fine particle.

### Reference Signs List

1 information processing system
2, 70 to 73 environmental measurement section
7 GPS (position-information acquisition section)
10 cellular phone (environmental measurement apparatus)
21, 121 laser source (light source)
25 light-receiving-element array
26, 126 diffraction grating
27 dichroic mirror (mirror)
28 total reflection mirror (mirror)
30 cloud server (information processing apparatus, another apparatus)
33 acquisition section
36 extraction section
37 information providing section
38 database building section
39 learning section
40 gas
80 database
141 fine particle
251a to 251i light receiving element

## Claims

1. An environmental measurement apparatus, comprising:
a light source that irradiates light onto a fine particle in gas; and
a light-receiving-element array that includes a plurality of light receiving elements receiving backscattered light obtained by the light being irradiated onto the fine particle.

2. The environmental measurement apparatus according to claim 1, further comprising:
a position-information acquisition section that acquires position information regarding a position of the environmental measurement apparatus; and
a communication section that communicates another apparatus, and transmits, to the other apparatus, measurement data measured by the light receiving element and the position information.

3. The environmental measurement apparatus according to claim 2, further comprising
a diffraction grating that the light from the light source enters, the diffraction grating emitting the light that is to be irradiated onto the fine particle.

4. The environmental measurement apparatus according to claim 2, further comprising
a mirror off which the backscattered light is reflected to enter the light-receiving-element array.

5. The environmental measurement apparatus according to claim 2, further comprising:
a plurality of the light-receiving-element arrays; and
a mirror through which light of a first wavelength from among the backscattered light is transmitted to enter one of the plurality of the light-receiving-element arrays, the mirror being a mirror off which light of a second wavelength that is different from the first wavelength is reflected to enter another of the plurality of the light-receiving-element arrays.

6. An information processing apparatus, comprising
an acquisition section that acquires measurement data of a fine particle in gas, and position information regarding a place in which the measurement data has been acquired, the measurement data and the position information being acquired by an environmental measurement section that includes a light source and a light-receiving-element array, the light source emitting light, the light-receiving-element array including a plurality of light receiving elements receiving backscattered light obtained by the emitted light being irradiated onto the fine particle.

7. The information processing apparatus according to claim 6, wherein
the acquisition section acquires the pieces of measurement data and the pieces of position information, the pieces of measurement data being respectively acquired by a plurality of the environmental measurement sections, the pieces of position information being respectively acquired by the plurality of the environmental measurement sections.

8. The information processing apparatus according to claim 7, further comprising
a learning section that performs statistical processing with respect to a relationship between the position information and the measurement data that are acquired from each of the plurality of the environmental measurement sections.

9. The information processing apparatus according to claim 8, wherein
the acquisition section acquires the measurement data of the fine particle and feeling information regarding a feeling of a user with respect to the fine particle upon acquiring the measurement data, and
the learning section performs statistical processing with respect to a relationship between the measurement data obtained from each of the plurality of the environmental measurement sections, and the feeling information.

10. The information processing apparatus according to claim 9, further comprising
a database building section that builds a database by accumulating therein the measurement data and position information regarding a region in which the measurement data has been acquired, the measurement data and the position information regarding the region being accumulated in a state of being associated with each other, the measurement data and the position information regarding the region being acquired by the environmental measurement section.

11. The information processing apparatus according to claim 10, wherein
the database building section accumulates therein information related to the fine particle, the measurement data, and the position information regarding the region in association with each other, the information related to the fine particle being generated on a basis of the measurement data and the position information regarding the region, and
the information processing apparatus further comprises an extraction section that refers to the database and extracts information related to a fine particle in gas in an arbitrary place selected by the user, the information related to the fine particle in the gas in the arbitrary place being associated with position information regarding the arbitrary place.

12. The information processing apparatus according to claim 11, further comprising
an information providing section that provides the information related to the fine particle in the gas in the arbitrary place, the information related to the fine particle in the gas in the arbitrary place being extracted by the extraction section.

13. The information processing apparatus according to claim 12, wherein
the information providing section provides the user with the information related to the fine particle in the gas in the arbitrary place, the information related to the fine particle in the gas in the arbitrary place being information in which the feeling information has been considered.

14. An information processing system, comprising:
a plurality of environmental measurement apparatuses each including a light source and a light-receiving-element array, the light source irradiating light onto a fine particle in gas, the light-receiving-element array including a plurality of light receiving elements receiving the light irradiated onto the fine particle; and
an acquisition section that acquires measurement data of the fine particle and position information regarding a place in which the measurement data has been acquired, the measurement data and the position information being acquired by each of the plurality of environmental measurement apparatuses.

15. An information processing method, comprising
acquiring a piece of measurement data of a fine particle in gas and a piece of position information regarding a place in which the piece of measurement data has been acquired, the pieces of measurement data and the pieces of position information being acquired by a plurality of environmental measurement sections each including a light source and a light-receiving-element array, the light source irradiating light onto a fine particle in gas, the light-receiving-element array including a plurality of light receiving elements receiving the light irradiated onto the fine particle.

16. A program that causes an information processing apparatus to perform a process comprising
acquiring a piece of measurement data and a piece of position information regarding a place in which the piece of measurement data has been acquired, the pieces of measurement data and the pieces of position information being acquired by a plurality of environmental measurement sections each including a light source and a light-receiving-element array, the light source irradiating light onto a fine particle in gas, the light-receiving-element array including a plurality of light receiving elements receiving the light irradiated onto the fine particle.
